# EUROPEAN PATENT SPECIFICATION

(11) **EP 4 217 509 B1**
(45) Date of publication and mention of the grant of the patent: **08.04.2026**
(21) Application number: 21786531.0
(22) Date of filing: 15.09.2021
(51) Int. Cl.: C12Q 1/6883

(54) **APPARATUS, KITS AND METHODS FOR PREDICTING THE DEVELOPMENT OF SEPSIS**
VORRICHTUNG, KITS UND VERFAHREN ZUR VORHERSAGE DER ENTWICKLUNG VON SEPSIS
APPAREILS, KITS ET PROCÉDÉS POUR PRÉDIRE LE DÉVELOPPEMENT D'UNE SEPTICÉMIE

(30) Priority: 22.09.2020 GR 20200100571
(43) Date of publication of application: 02.08.2023
(73) Proprietor: THE SECRETARY OF STATE FOR DEFENCE, Salisbury, Wiltshire SP4 0JQ (GB)
(72) Inventor: LUKASZEWSKI, Roman Antoni, Salisbury, Wiltshire SP4 0JQ (GB); PANAGIOTOU, loannis, 10405 Berlin (DE); SCHMIDT-HECK, Wolfgang, 07749 Jena (DE)
(74) Representative: Phillips, Thomas Edward
(86) International application number: PCT/GB2021/000103
(87) International publication number: WO 2022/064164

(56) References cited:
- WO-A1-2015/121605
- WO-A2-2006/113529
- GAVIN C. K. W. KOH ET AL: "Host Responses to Melioidosis and Tuberculosis Are Both Dominated by Interferon-Mediated Signaling", PLOS ONE, vol. 8, no. 1, 1 January 2013 (2013-01-01), pages e54961, XP055376146, DOI: 10.1371/journal.pone.0054961
- ILLUMINA: "HumanHT-12 v4 BeadChip Product Information", 1 January 2010 (2010-01-01), pages 1 - 2, XP055178072, Retrieved from the Internet <URL:http://www.illumina.com/documents/products/product_information_sheets/product_info_humanht-12.pdf> [retrieved on 20150320]
- AMBION: "Illumina TotalPrep TM RNA Amplification Kit", 1 January 2011 (2011-01-01), pages 1 - 30, XP055178075, Retrieved from the Internet <URL:http://tools.lifetechnologies.com/content/sfs/manuals/IL1791ME.pdf> [retrieved on 20150320]

## Description

The present invention is concerned with kits, methods and apparatus for analysing a biological sample from a subject to predict and/or monitor the development of infection and/or organ dysfunction and/or sepsis utilising groups of nucleic acid markers to predict the development of infection and/or organ dysfunction and/or sepsis.

Following exposure to a microbial pathogen there is often a lag phase before symptoms of infection, which could further result in symptoms of organ dysfunction, and development of sepsis. After the onset of clinical symptoms, the effectiveness of treatment often decreases as the disease progresses, so the time taken to make any diagnosis is critical. It is likely that a detection or diagnostic assay will be the first confirmed indicator of infection, organ dysfunction or sepsis. The availability, rapidity and predictive accuracy of such an assay will therefore be crucial in determining the outcome. Any time saved will speed up the implementation of medical countermeasures and will have a significant impact on recovery.

The development of technologies to facilitate rapid detection of infection, organ dysfunction and sepsis is a key concern for all at risk. During the initial stages of infection many biological agents are either absent from, or present at very low concentrations in, typical clinical samples (e.g. blood). It is therefore likely that agent-specific assays would have limited utility in detecting infection before clinical symptoms arise. Previous studies have shown that infection elicits a pattern of immune response involving changes in the expression of a variety of biomarkers that is indicative of the type of agent. Such patterns of biomarker expression have proven to be diagnostic for a variety of infectious agents. It is now possible to distinguish patterns of gene expression in blood leukocytes from symptomatic patients with acute infections caused by four common human pathogens (Influenza A, *Staphylococcus aureus, Streptococcus pneumoniae* and *Escherichia coli)* using whole transcriptome analysis. More recently, researchers have been able to reduce the number of host biomarkers required to make a diagnosis through use of appropriate bioinformatic analysis techniques to select key biomarkers for the diagnosis of infectious disease.

While host biomarker signatures represent an attractive solution for the prediction of microbial infection, their discovery relies on the exploitation of laboratory models of infection whose fidelity to the pathogenesis of disease in humans varies. An alternative approach for biomarker discovery in humans is to exploit a common sequela of biological agent infection; such as the life-threatening condition sepsis, which now requires organ dysfunction for a positive diagnosis. Sepsis has traditionally been defined as a systemic inflammatory response syndrome (SIRS) in response to infection which, when associated with acute organ dysfunction, may ultimately cause severe life-threatening complications. However, sepsis is now defined as life-threatening organ dysfunction caused by a dysregulated host response to infection, wherein organ dysfunction can be identified as an increase in the total sequential organ failure assessment (SOFA) score of 2 or more points from one day to the next following infection. Severity of organ dysfunction has in the past been assessed with various scoring systems that quantify abnormalities according to clinical findings, laboratory data, or therapeutic interventions. The predominant score in current use is the SOFA (originally the Sepsisrelated Organ Failure Assessment). A higher SOFA score is associated with an increased probability of mortality. The score grades abnormality by organ system and accounts for clinical interventions. The baseline SOFA score can be assumed to be zero in patients not known to have pre-existing organ dysfunction. A SOFA score ≥2 reflects an overall mortality risk of approximately 10% in a general hospital population with suspected infection.

Sepsis is a major cause of morbidity and mortality in intensive care units (ICU). In the UK, sepsis is believed to be responsible for about 27% of all ICU admissions. Across Europe the average incidence of sepsis in the ICU is about 30%, with a mortality rate of 27%. In the USA, hospital-associated mortality from sepsis ranges between 18 to 30%; an estimated 9.3% of all deaths occurred in patients with sepsis. Clearly there is a very accessible patient population that could be used to study predictive markers for the onset of sepsis.

Despite greatly improved diagnosis, treatment and support, serious infection and sepsis remain significant causes of death and often result in chronic ill-health or disability in those who survive acute episodes. Although sudden, overwhelming infection is comparatively rare amongst otherwise healthy adults, it constitutes an increased risk in immunocompromised individuals, seriously ill patients in intensive care, burns patients and young children. In a proportion of cases, an apparently treatable infection leads to the development of sepsis; a dysregulated, inappropriate response to infection characterised by progressive circulatory collapse leading to renal and respiratory failure, abnormalities in coagulation, profound and unresponsive hypotension and, in about 30% of cases death. The incidence of sepsis in the population of North America is about 0.3% of the population annually (about 750,000 cases) with mortality rising to 40% in the elderly and to 50% in cases of the most severe form, septic shock.

The ability to detect potentially serious infections and organ dysfunction as early as possible and, especially, to predict the onset of sepsis in susceptible individuals is clearly advantageous.

Although a number of biomarkers (markers), such as nucleic acid markers or protein markers, have been shown to correlate with sepsis and some give an indication of the seriousness of the condition, no single marker or combination of markers has yet been shown to be a reliable diagnostic test, much less a predictor of the development of sepsis, especially sepsis meeting the criteria of the new definition, requiring life-threatening organ dysfunction.

Extracting reliable diagnostic patterns and robust prognostic indications from changes over time in complex sets of variables including traditional clinical observations, clinical chemistry, biochemical, immunological and cytometric data requires sophisticated methods of analysis. The use of expert systems and artificial intelligence, including neural networks, for medical diagnostic applications has been being developed for some time.

The ability to detect the earliest signs of infection and/or organ dysfunction and/or sepsis has clear benefits in terms of allowing treatment as soon as possible. Indications of the severity of the condition and likely outcome if untreated inform decisions about treatment options. This is relevant both in vulnerable hospital populations, such as those in intensive care, or who are burned or immunocompromised, and in other groups in which there is an increased risk of serious infection and subsequent sepsis. The use or suspected use of biological weapons in both battlefield and civilian settings is an example where a rapid and reliable means of testing for the earliest signs of infection or organ dysfunction (i.e. sepsis) in individuals exposed would also be advantageous. However, until now the majority of investigations focussed on developing a group of biomarkers and/or a test for sepsis were based on the previous definition of sepsis of systemic inflammatory response syndrome (SIRS) in response to infection, and thus have generally focussed on identifying a group of biomarkers and/or a test to predict SIRS in response to infection. Sepsis is however now defined as life-threatening organ dysfunction caused by a dysregulated host response to infection, and thus a group of biomarkers and/or a test is required which is capable of identifying individuals at risk of developing life-threatening organ dysfunction caused by a dysregulated host response to infection. Until now neither a test nor a list of biomarkers has been identified/produced which can detect or predict sepsis (organ dysfunction) with a good/high predictive accuracy (for example with an area under the curve (AUC) > 0.8, but preferably > 0.85, and most preferably > 0.9). WO 2015/121605 A1 (2015-08-20) identifies a specific list of 25 biomarkers that is capable of a predictive accuracy of at least 95% to differentiate development of sepsis from SIRS.

The present invention thus aims to provide biomarker signatures (groups of biomarkers), and methods for classifying biological samples using the biomarker signatures, to predict/detect the development of organ dysfunction, and consequently sepsis, and preferably both infection and organ dysfunction, and sepsis, with a high predictive accuracy. The invention is defined in the appended claims.

With this in mind, the applicants have determined several lists/groups (signatures) of nucleic acid markers (biomarkers) which can be used to predict the development of infection and organ dysfunction, and thus predict the development of sepsis, with the possibility of predicting prior to the onset of symptoms (pre-symptomatic). The Applicant has identified through a comprehensive analysis of the host transcriptome, sourced from blood samples from human patients collected prior to the clinical onset of sepsis, a panel of 80 nucleic acids highly significant to predicting sepsis, with an ability to predict both infection and organ dysfunction, and subsets thereof for predicting development of infection and/or organ dysfunction and/or sepsis.

The 80 nucleic acid markers highly significant to predicting infection and organ dysfunction are:
ACTR6, AFF1, AKR1B1, ARIDSB, ASNSD1, ATP2A2, ATXN1, ATXN7L3, B3GNT5, B4GALT5, BIN1, BIRCS, C11ORF1, C19ORF70, CAPN15, CD247, CD3D, CD3E, CFLAR, CPA3, CR1, CTDP1, CTSS, DENND4B, DHRS3, DOK3, EIF4G3, FBXW2, FCER1A, FKBP5, GALM, GAS7, GPR183, GRB10, GZMK, HLA-DMA, HLA-DMB, HLA-DOA, HVCN1, ICAM2, IL1R1, INO80D, KIF1B, KLHL2, LARP4B, LDHA, LDLR, LEPROTL1, LETMD1, LGALS2, LSG1, MAFG, MED13L, METTL7B, MIDN, MRPS27, NFKBIA, NME8, P4HB, QSOX1, RARRES3, RPL13A, RPS10, RPS13, RPS14, SGSH, SLC26A6, SLC2A11, SLC36A1, SLC41A3, SOS2, SPATA13, STOM, TCEA3, TLR2, TNFAIP3, TRPM2, ZKSCAN1, ZNF195, ZNF608.

Details of the particular nucleic acids, from their annotated names (as provided throughout this application), can be found from human gene databases such as GeneCards^{®}: The Human Gene Database (genecards.org), or the Human Gene Resources at the National Center for Biotechnology Information (NCBI) (ncbi.nlm.nih.gov/genome/guide/human/).

Organ dysfunction and infection are two key indicators of sepsis, and thus there is a need to identify markers which are capable of predicting development of either, though at least organ dysfunction, but preferably both, with good levels of predictivity, and as early as possible, and preferably before symptoms occur. The Applicant has down-selected lists of nucleic acid markers, as detailed above, critical to predicting sepsis with a high level of confidence in the prediction, which are capable of providing a prediction up to at least three days in advance of symptoms.

Subsets of the group of 80 nucleic acid markers, in particular subsets from as few as 4 biomarkers, have been shown to be capable of predicting development of infection versus absence of infection (and development of infection vs development of SIRS), and predicting development of organ dysfunction versus non-complicated infection and/or absence of organ dysfunction, and thus predict sepsis versus absence of sepsis, through analysis of samples from subjects (for example patients) categorised as progressing to development of sepsis, or not, or as progressing to having an infection, or not.

Thus in a first aspect the present description, that is not part of the invention, provides a method for predicting the development of infection and/or organ dysfunction and/or sepsis in a subject, the method comprising determining levels of at least four nucleic acid markers, or a product expressed by those nucleic acids, such as the corresponding proteins, in a biological sample taken from the subject, wherein the at least four nucleic acid markers are selected from the list consisting of:
ACTR6, AFF1, AKR1B1, ARID5B, ASNSD1, ATP2A2, ATXN1, ATXN7L3, B3GNT5, B4GALT5, BIN1, BIRCS, C11ORF1, C19ORF70, CAPN15, CD247, CD3D, CD3E, CFLAR, CPA3, CR1, CTDP1, CTSS, DENND4B, DHRS3, DOK3, EIF4G3, FBXW2, FCER1A, FKBP5, GALM, GAS7, GPR183, GRB10, GZMK, HLA-DMA, HLA-DMB, HLA-DOA, HVCN1, ICAM2, IL1R1, INO80D, KIF1B, KLHL2, LARP4B, LDHA, LDLR, LEPROTL1, LETMD1, LGALS2, LSG1, MAFG, MED13L, METTL7B, MIDN, MRPS27, NFKBIA, NME8, P4HB, QSOX1, RARRES3, RPL13A, RPS10, RPS13, RPS14, SGSH, SLC26A6, SLC2A11, SLC36A1, SLC41A3, SOS2, SPATA13, STOM, TCEA3, TLR2, TNFAIP3, TRPM2, ZKSCAN1, ZNF195, ZNF608;
wherein the levels of the at least four nucleic acid markers, or the products expressed by those nucleic acids, are used to predict the development of infection and/or organ dysfunction and/or sepsis.

The levels of the nucleic acid markers, or the products expressed by those nucleic acids, are preferably collectively, and in combination, used to predict the development of infection and/or organ dysfunction and/or sepsis, which could be through use of a mathematical model applied to the levels of the nucleic acid markers, or the products expressed by those nucleic acids, to provide the prediction.

The method of the first aspect may comprise use of a control in the method from which to establish the level (or expression level) of each nucleic acid, or a product thereof. The control may for example be one or more housekeeping genes/nucleic acids whose expression is predictable or relatively static irrespective of whether infection and/or organ dysfunction may develop. For example, the reference gene/nucleic acid may be FAM105B and/or RANBP3.

The term 'biological sample' includes, but not exclusively, blood, serum, plasma, urine, saliva, cerebrospinal fluid or any other form of material, preferably fluid-based or capable of being converted into a fluid-like state (e.g. tissue which can be broken down or separated in a solution, such as a buffered solution), which can be extracted or collected from a patient.

For prediction of infection versus absence of infection the Applicant has in particular identified 47 markers (through microarray and q-PCR analysis), from the list of 80 nucleic acid markers which are significant to a prediction, with selections (signatures) of at least 4 or at least 5, though optimally at least 7 or at least 8, markers from the 47 being particularly effective for providing a prediction of infection versus an absence of infection, with a high level of predictivity.

The group of 47 markers is: ACTR6, AFF1, ARID5B, ASNSD1, ATP2A2, ATXN7L3, B4GALT5, BIRCS, C11ORF1, CAPN15, CD247, CD3E, CR1, CTDP1, DOK3, EIF4G3, FKBP5, GALM, GAS7, GPR183, HLA-DMA, HLA-DMB, HLA-DOA, HVCN1, KIF1B, KLHL2, LARP4B, LDHA, LDLR, LEPROTL1, LETMD1, LGALS2, LSG1, MAFG, METTL7B, NFKBIA, P4HB, RPS10, SLC26A6, SLC2A11, SLC36A1, SOS2, TCEA3, TNFAIP3, TRPM2, ZKSCAN1, ZNF195.

For example, the following subsets from the list of 47 markers are capable of achieving an AUC > 0.9 for predicting development of infection on specific days prior to development of symptoms:
1. ASNSD1, B4GALT5, BIRC5, C11ORF1, CR1, KIF1B, LDLR, ZKSCAN1 (Day-1 before symptoms)
2. ARIDSB, B4GALT5, CD3E, CTDP1, HVCN1, P4HB, SLC2A11, ZNF195 (Day-2 before symptoms)
3. B4GALT5, CTDP1, DOK3, GPR183, LDLR, NFKBIA, P4HB, SOS2 (Day-3 before symptoms)
4. ATP2A2, GALM, HLA-DMA, HLA-DOA, LDLR, SLC36A1, TRPM2 (Day-1 before symptoms)
5. ACTR6, CAPN15, CD247, HLA-DMB, LARP4B, LDLR, LGALS2, LSG1, METTL7B, TNFAIP3 (Day-2 before symptoms)
6. AFF1, CAPN15, FKBP5, GALM, KLHL2, LDHA, LDLR, LEPROTL1, LETMD1, MAFG, RPS10, TCEA3 (Day-3 before symptoms).

These subsets of markers may be optimal for a specific day before the onset of symptoms of infection, or sepsis, although may be used in a test to identify development of infection or sepsis at any point before or after onset of symptoms, whereas other subsets of markers from the list of 47 markers may also be used for providing predictions, on individual days before symptoms or multiple days before symptoms, for example other subsets of markers from the list of 47 may be capable of predicting development of infection (or sepsis) with an AUC > 0.9 irrespective of the number of days prior to onset of symptoms, up to at least three days prior to onset of symptoms. For example, the 8 marker subset of LDLR, B4GALT5, KIF1B, CTDP1, GAS7, SLC26A6, HLA-DMB, EIF4G3, or the 7 marker subset of B4GALT5, AFF1, LDLR, ATXN7L3, LARP4B, SLC36A1, TRPM2.

Key nucleic acid markers, present in multiple (2 or more) down-selected subsets for prediction of infection versus no-infection are, from microarray analysis, B4GALT5, CTDP1, LDLR, KIF1B, P4HB, and especially B4GALT5, CTDP1, LDLR, which are present in at least 3 or 4 different subsets.

Key nucleic acid markers, present in multiple (2 or more) down-selected subsets for prediction of infection versus no-infection are, from q-PCR analysis, LDLR, AFF1, CAPN15, GALM, LARP4B, SLC36A1, ZKSCAN1, and especially LDLR, which is present in at least 4 different subsets.

The selection of markers thus may comprise one or more of the key nucleic acid markers that recur during down-selection of subsets of markers. A subset may for example comprise B4GALT5, CTDP1, and LDLR, markers that recur in numerous subsets, and may in addition, or substitution, comprise KIF1B, P4HB, AFF1, CAPN15, GALM, LARP4B, SLC36A1, or ZKSCAN1.

For prediction of development of infection vs development of SIRS the Applicant has in particular identified 19 markers from the list of 80 nucleic acid markers significant to predicting development of infection vs development of SIRS, with selections of at least 4, though optimally at least 8, from the 19 being particularly effective for providing a prediction of development of infection vs development of SIRS.

The group of 19 markers is: ATXN1, B4GALT5, CFLAR, DENND4B, EIF4G3, FBXW2, FKBP5, GZMK, HVCN1, LDLR, MED13L, METTL7B, MIDN, NME8, RPS14, SLC36A1, SLC41A3, SPATA13, STOM.

For example, the following subsets from the list of 19 markers are capable of achieving an AUC > 0.9 for predicting development of infection on specific days prior to development of symptoms:
CFLAR, DENND4B, EIF4G3, FXBP5, GZMK, LDLR, MED13L, NME8, RPS14, SLC36A1, SLC41A3, SPATA13 (Day-1 before symptoms)
CFLAR, FKBP5, HVCN1, LDLR, NME8, SLC41A3, SPATA13, STOM (Day-2 before symptoms)
CFLAR, DENND4B, EIF4G3, FBXW2, MED13L, METTL7B, SPATA13, STOM (day-3 before symptoms)

These subsets of markers may be optimal for a specific day before the onset of symptoms of infection, or sepsis, although may be used in a test to identify development of infection or sepsis at any point before or after onset of symptoms, whereas other subsets of markers from the list of 19 markers may also be used for providing predictions, on individual days before symptoms or multiple days before symptoms, for example other subsets of markers from the list of 47 may be capable of predicting development of infection versus development of SIRS with an AUC > 0.9, irrespective of the number of days prior to onset of symptoms, up to at least three days prior to onset of symptoms. For example, the 12 marker subset of ATXN1, B4GALT5, CFLAR, EIF4G3, HVCN1, LDLR, MED13L, METTL7B, MIDN, SLC41A3, SPATA13, STOM.

Key nucleic acid markers, present in multiple (2 or more) down-selected subsets for prediction of infection versus development of SIRS are, from q-PCR analysis, CFLAR, DENND4B, EIF4G3, FKBP5, HVCN1, LDLR, MED13L, METTL7B, NME8, SLC41A3, SPATA13, STOM, and especially CFLAR, EIF4G3, LDLR, MED13L, SLC41A3, SPATA13, STOM which are present in at least 3 or 4 different subsets.

The selection of markers thus may comprise one or more of the key nucleic acid markers that recur during down-selection of subsets of markers. A subset may for example comprise one or more of CFLAR, EIF4G3, LDLR, MED13L, SLC41A3, SPATA13, and STOM, markers that recur in numerous subsets.

For prediction of organ dysfunction versus absence of organ dysfunction the Applicant has in particular identified 45 markers which are significant to a prediction, with selections of at least 4, though optimally at least 7 or 8, from the 45 being particularly effective for providing a prediction of organ dysfunction versus absence of organ dysfunction with a high level of predictivity.

The group of 45 markers is: AFF1, AKR1B1, B3GNT5, BIN1, BIRCS, C11ORF1, C19ORF70, CD247, CD3D, CD3E, CPA3, CTSS, DHRS3, DOK3, FCER1A, GAS7, GRB10, HLA-DMA, HLA-DMB, ICAM2, IL1R1, INO80D, KIF1B, LARP4B, LDLR, LGALS2, LSG1, MED13L, METTL7B, MIDN, MRPS27, P4HB, QSOX1, RARRES3, RPL13A, RPS13, RPS14, SGSH, SLC36A1, SOS2, STOM, TCEA3, TLR2, TNFAIP3, ZNF608.

For example, the following subsets from the list of 45 markers are capable of achieving an AUC > 0.85 for predicting development of organ dysfunction on specific days prior to development of symptoms:
1. HLA-DMA, HLA-DMB, ICAM2, INO80D, KIF1B, MED13L, QSOX1 (Day-1 before symptoms)
2. AFF1, BIN1, C19ORF70, GAS7, LDLR, MIDN, MRPS27, P4HB (Day-2 before symptoms)
3. C11ORF1, CD3D, CTSS, LSG1, RPL13A (Day-3 before symptoms)
4. AKR1B1, DOK3, ICAM2, IL1R1, RPL13A, RPS14, SGSH, TLR2 (Day-1 before symptoms)
5. B3GNT5, C11ORF1, CD3E, IL1R1, LARP4B, LGALS2, LSG1, METTL7B, P4HB, SLC36A1, SOS2, STOM (Day-2 before symptoms)
6. CD247, CD3D, FCER1A, GRB10, IL1R1, LGALS2, RARRES3, RPS14, SGSH, TCEA3 (Day-3 before symptoms)

These subsets of markers may be optimal for a specific day before the onset of symptoms of organ dysfunction (sepsis), although may be used in a test to identify development of infection or sepsis at any point before or after onset of symptoms, whereas other subsets of markers from the list of 45 markers may also be used for providing predictions, on individual days before symptoms or multiple days before symptoms, for example other subsets of markers from the list of 47 may be capable of predicting development of infection with an AUC > 0.85, irrespective of the number of days prior to onset of symptoms, up to at least three days prior to onset of symptoms. For example, the 8 marker subset of BIRC5, CPA3, DHRS3, HLA-DMA, ICAM2, MIDN, TNFAIP3, ZNF608, or the 8 marker subset of SGSH, RPS13, DOK3, ICAM2, IL1R1, LGALS2, LSG1, RPL13A.

Key nucleic acid markers, present in multiple (2 or more) down-selected subsets for prediction of organ dysfunction versus no organ dysfunction are, from microarray analysis, HLA-DMA, ICAM2, and MIDN.

Key nucleic acid markers, present in multiple (2 or more) down-selected subsets for prediction of organ dysfunction versus no organ dysfunction are, from q-PCR analysis, IL1R1, LGALS2, SGSH, DOK3, ICAM2, LSG1, RPL13A, RPS14, and especially IL1R1, LGALS2, SGSH which are present in at least 3 or 4 different subsets.

The selection of markers thus may comprise one or more of the key nucleic acid markers that recur during down-selection of subsets of markers. A subset may for example comprise IL1R1, LGALS2, and SGSH, markers that recur in numerous subsets, and may in addition, or substitution, comprise DOK3, ICAM2, LSG1, RPL13A, RPS14, HLA-DMA or MIDN.

The biomarker signatures (lists of nucleic acid markers) for predicting development of organ dysfunction are all capable of achieving an AUC of at least 0.75, with the majority achieving an AUC of > 0.8 or > 0.85, which is particularly advantageous for predicting organ dysfunction, which until now has been difficult to predict, with the majority of studies in the past focussing on predicting sepsis based on the previous definition of sepsis, i.e. SIRS in response to infection, thus infection versus absence of infection alone.

For prediction of organ dysfunction versus all other clinical symptoms, the Applicant has identified a number of nucleic acid marker sets, or nucleic acid biomarker signatures, which are significant to a prediction of organ dysfunction (versus patient samples with any other clinical symptoms, or indeed non-symptomatic controls, but critically without symptoms of organ dysfunction) with a high level of predictivity.

For example, the following biomarker signatures are capable of achieving an AUC > 0.80 or an AUC >0.85 for predicting development of organ dysfunction irrespective of the number of days prior to onset of symptoms, up to at least three days prior to onset of symptoms:
1. AFF1, ATXN1, ATXN7L3, B4GALT5, CFLAR, HVCN1, LDLR, LGALS2, LSG1, MED13L, RPS13, SGSH, SLC36A1, ICAM2, MIDN, METTL7B, LARP4B, EIF4G3, STOM, TRPM2, RPL13A, DOK3.
2. AFF1, ATXN1, ATXN7L3, B4GALT5, CFLAR, HVCN1, LDLR, LGALS2, LSG1, MED13L, SGSH, MIDN, METTL7B, LARP4B.
3. ATXN7L3, B4GALT5, LDLR, MED13L, ATXN1, CFLAR
4. ATXN7L3, B4GALT5, LDLR, MED13L.
5. AFF1, ATXN1, ATXN7L3, B4GALT5, CFLAR, HVCN1, LDLR, LGALS2, LSG1, MED13L, RPS13, SGSH, SLC36A1.

The biomarker signatures of the present invention are thus especially valuable as they are capable of providing a test that can predict both infection and organ dysfunction, either together or separately, as part of a test for predicting sepsis, with the added value of better informing treatment and monitoring treatment of a subject.

The at least 4 nucleic acid markers to be determined in the method of the first aspect of the invention may be selected from any of the biomarker signatures, subsets, or key nucleic acid markers, that have been identified or down-selected for differentiating development of infection from no-infection, development of infection from development or SIRS, development of organ dysfunction from non-complicated infection or no-organ dysfunction, or development of organ dysfunction versus all other clinical symptoms (or non-symptomatic controls), and in any combination, from the list of 80 markers identified as highly significant to predicting sepsis.

In a second aspect, the present description, that is not part of the invention, provides a method for monitoring a subject at risk of developing infection and/or organ dysfunction and/or sepsis, the method comprising determining levels of at least four nucleic acid markers, or the products expressed by those nucleic acids, in biological samples taken from a subject at multiple time points, wherein the monitored levels of the at least four markers are used to predict development of infection and/or organ dysfunction and/or sepsis, wherein the at least four nucleic acid markers are selected from the list consisting of:
ACTR6, AFF1, AKR1B1, ARIDSB, ASNSD1, ATP2A2, ATXN1, ATXN7L3, B3GNT5, B4GALT5, BIN1, BIRCS, C11ORF1, C19ORF70, CAPN15, CD247, CD3D, CD3E, CFLAR, CPA3, CR1, CTDP1, CTSS, DENND4B, DHRS3, DOK3, EIF4G3, FBXW2, FCER1A, FKBP5, GALM, GAS7, GPR183, GRB10, GZMK, HLA-DMA, HLA-DMB, HLA-DOA, HVCN1, ICAM2, IL1R1, INO80D, KIF1B, KLHL2, LARP4B, LDHA, LDLR, LEPROTL1, LETMD1, LGALS2, LSG1, MAFG, MED13L, METTL7B, MIDN, MRPS27, NFKBIA, NME8, P4HB, QSOX1, RARRES3, RPL13A, RPS10, RPS13, RPS14, SGSH, SLC26A6, SLC2A11, SLC36A1, SLC41A3, SOS2, SPATA13, STOM, TCEA3, TLR2, TNFAIP3, TRPM2, ZKSCAN1, ZNF195, ZNF608.

The biomarkers of the present description, or subsets thereof, are particularly advantageous for use in a test for monitoring a subject at risk over several days, which test could incorporate different marker sets to identify the most likely day before symptoms, such as Day-1, Day-2, or Day-3 before symptoms, and whether infection and/or organ dysfunction is likely, and thereby consequently inform the best course of treatment to prevent or treat infection/organ dysfunction. The monitoring may comprise comparing dynamic changes in quantitation or rates of change of the biomarkers to derive predictors, such as for whether and when a subject may develop sepsis. For example, monitoring may comprise interrogation of biomarker velocity, such as its rate of change over time. An AUC of 0.8 and above for predicting organ dysfunction is achievable with nucleic acid markers subsets form the list of 80 markers, which is particularly good for predicting organ dysfunction versus absence of organ dysfunction, as no biomarker set to date has been identified that is particularly directed to organ dysfunction, since most studies on sepsis have used the previous definition of sepsis, which was concerned with infection alone, and not organ dysfunction, and especially not an approach that could evaluate both infection and organ dysfunction with a single set of biomarkers, or different groups of biomarkers for each of infection and organ dysfunction. Particular subsets of markers are as for the first aspect of the present invention.

In a third aspect, the present description, that is not part of the invention, provides a kit for predicting development of infection and/or organ dysfunction and/or sepsis in a subject, said kit comprising reagents and/or systems for determining levels of at least four markers in a biological sample from a subject, wherein the at least four markers are selected from the list consisting of:
ACTR6, AFF1, AKR1B1, ARIDSB, ASNSD1, ATP2A2, ATXN1, ATXN7L3, B3GNT5, B4GALT5, BIN1, BIRCS, C11ORF1, C19ORF70, CAPN15, CD247, CD3D, CD3E, CFLAR, CPA3, CR1, CTDP1, CTSS, DENND4B, DHRS3, DOK3, EIF4G3, FBXW2, FCER1A, FKBP5, GALM, GAS7, GPR183, GRB10, GZMK, HLA-DMA, HLA-DMB, HLA-DOA, HVCN1, ICAM2, IL1R1, INO80D, KIF1B, KLHL2, LARP4B, LDHA, LDLR, LEPROTL1, LETMD1, LGALS2, LSG1, MAFG, MED13L, METTL7B, MIDN, MRPS27, NFKBIA, NME8, P4HB, QSOX1, RARRES3, RPL13A, RPS10, RPS13, RPS14, SGSH, SLC26A6, SLC2A11, SLC36A1, SLC41A3, SOS2, SPATA13, STOM, TCEA3, TLR2, TNFAIP3, TRPM2, ZKSCAN1, ZNF195, ZNF608.

The subject is most likely a human, but may also be an animal, and the biological sample is most likely a blood or serum sample.

Particular subsets of markers are as for the first aspect of the present invention.

The kit of the invention may comprise means for detecting levels of a nucleic acid or nucleic acid product. Although nucleic acid expression may be determined by detecting the presence of nucleic acid products including proteins and peptides, such processes may be complex. In a particular embodiment, the means comprises means for detecting a nucleic acid, for example RNA, such as mRNA, or means for detecting a product expressed by the nucleic acid, such as a protein.

The reagents or systems may include use of recognition elements, or microarray based methods. Thus in a particular embodiment, the kit of the invention comprises a microarray on which are immobilised probes suitable for binding to RNA expressed by each nucleic acid of a biomarker signature. Means for detecting a protein may comprise an antibody, which may be a fluorescently-labelled antibody, and may comprise protein recognition elements on a microarray, or other suitable platform, such as lateral flow strips.

In an alternative embodiment, the kit comprises at least some of the reagents suitable for carrying out amplification of nucleic acids of the biomarker signature, or regions thereof.

In one embodiment the reagents or systems use real-time (RT) polymerase chain reaction (PCR). In such cases, the reagents may comprise primers for amplification of said nucleic acids or regions thereof. The kits may further comprise labels, in particular fluorescent labels, and/or oligonucleotide probes to allow the PCR to be monitored in real-time using any of the known assays, such as TaqMan, LUX, etc. The kits may also contain reagents such as buffers, enzymes, salts such as MgCl etc. required for carrying out a nucleic acid amplification reaction. The reagents, especially for nucleic acid amplification, may comprise for example one or more of fluorescently-labelled oligonucleotide probes or fluorescently-labelled primers, wherein the fluorescently-labelled oligonucleotide probes or fluorescently-labelled primers may consist of probes and primers each capable of specific binding and detection of nucleic acid products of the at least 4 markers.

The methods of the first or second aspect may advantageously be computer-implemented to handle the complexity in monitoring and analysis of the numerous biomarkers, and their respective relationships to each other. Such a computer-implemented invention could enable a yes/no answer as to whether infection and/or organ dysfunction and/or sepsis is likely to develop, or at least provide an indication of how likely the development is.

The method preferably uses mathematical tools and/or algorithms to monitor and assess the (expression) levels of the biomarkers (the nucleic acid markers, or products thereof) both qualitatively and quantitatively. The tools could in particular include support vector machine (SVM) algorithms, decision trees, random forests, artificial neural networks, quadratic discriminant analysis, and Bayes classifiers. In one embodiment the data from monitoring all biomarkers in the biomarker signature is assessed by means of an artificial neural network.

In one embodiment of the first or second aspect the method is a computer-implemented method wherein the monitoring, measuring and/or detecting comprises producing quantitative, and optionally qualitative, data for all markers, inputting said data into an analytical process on the computer, using at least one mathematical method, that may compare the data with reference data, and producing an output from the analytical process which provides a prediction for the likelihood of developing infection and/or organ dysfunction and/or sepsis, or enables monitoring of the condition. The reference data may include data from healthy subjects, subjects diagnosed with sepsis (organ dysfunction), subjects with infection, and subjects with SIRS, but no infection.

The output from the analytical process may enable the time to onset of symptoms to be predicted, such as 1, 2, or 3 days prior to onset of symptoms, and consequently may be particularly valuable and useful to a medical practitioner in suggesting a course of treatment, especially when the choice of course of treatment is dependent on the progression of the disease. The method may also enable monitoring of the success of any treatment, assessing whether the likelihood of onset of symptoms decreases over the course of treatment.

In a fourth aspect, the present description, that is not part of the invention, provides an apparatus for analysis of a biological sample from a subject to predict or monitor the development of sepsis comprising means for monitoring, measuring or detecting the expression of at least four markers in a biological sample from the subject, wherein the at least four markers are selected from the list consisting of:
ACTR6, AFF1, AKR1B1, ARID5B, ASNSD1, ATP2A2, ATXN1, ATXN7L3, B3GNT5, B4GALT5, BIN1, BIRCS, C11ORF1, C19ORF70, CAPN15, CD247, CD3D, CD3E, CFLAR, CPA3, CR1, CTDP1, CTSS, DENND4B, DHRS3, DOK3, EIF4G3, FBXW2, FCER1A, FKBP5, GALM, GAS7, GPR183, GRB10, GZMK, HLA-DMA, HLA-DMB, HLA-DOA, HVCN1, ICAM2, IL1R1, INO80D, KIF1B, KLHL2, LARP4B, LDHA, LDLR, LEPROTL1, LETMD1, LGALS2, LSG1, MAFG, MED13L, METTL7B, MIDN, MRPS27, NFKBIA, NME8, P4HB, QSOX1, RARRES3, RPL13A, RPS10, RPS13, RPS14, SGSH, SLC26A6, SLC2A11, SLC36A1, SLC41A3, SOS2, SPATA13, STOM, TCEA3, TLR2, TNFAIP3, TRPM2, ZKSCAN1, ZNF195, ZNF608;
and means for analysis of data produced from the means for monitoring, measuring or detecting, such as a computer comprising an appropriate mathematical model to analyse the data, such as an artificial neural network, and means for providing an output from the analysis which output provides a prediction of the likelihood of a subject having sepsis, or an output to enable monitoring of infection and/or organ dysfunction and/or sepsis, which output could also be provided by an appropriately programmed computer.

All subsets of markers (biomarker signatures) of the different aspects of the present invention may be used to monitor and/or predict the response of a subject to a particular therapeutic agent, such as a sepsis targeting drug or an antibiotic. For example the expression of particular nucleic acid markers, which may for example be elevated in a subject on a course to develop sepsis, or having already developed sepsis, could be monitored to establish whether the levels are returning to the levels expected for a subject without, or unlikely to develop, sepsis, which could be an indication of the therapeutic agent successfully treating the subject. A therapeutic agent may be one targeted to particular subsets of markers in an attempt to treat the subject, and indeed the choice of therapeutic agent to be used in a subject may be determined by the expression of specific nucleic acid markers which are most affected, or differ most from a control or from a patient not predicted to develop sepsis, as a result of developing sepsis. For example, the elevation of certain markers may suggest the use of one therapeutic agent, whereas elevation of a different subset of markers, may suggest use of another therapeutic agent.

Any feature in one aspect of the invention may be applied to any other aspects of the invention, in any appropriate combination. In particular, method aspects may be applied to use, kit and system aspects and vice versa. The invention extends to methods, uses, kits or systems substantially as herein described, with reference to the Example(s).

Example - Development of a predictive panel of pre-symptomatic biomarkers for infection, organ dysfunction, and thereby sepsis

The aim of this program of work was to develop a predictive panel of pre-symptomatic biomarkers for infection and organ dysfunction (sepsis), through comprehensive analysis of the host transcriptome, sourced from blood samples from human patients collected prior to the clinical onset of infection or organ dysfunction, and to develop biomarker signatures that may indicate whether and when clinical symptoms will arise. In so doing it would yield a suitably powered bioinformatic model for differentiating patients developing sepsis from those that were unlikely to develop sepsis based on transcriptomic biomarker signatures. In turn, this will assist in the development of (RT-PCR) methods for infection and organ dysfunction prediction, where this capability should provide timely diagnosis and treatment when medical countermeasures are most effective.

We used microarray technology to obtain gene (nucleic acid) expression data of samples derived from pre-symptomatic patients and control patient samples. An unsupervised bioinformatic approach was used to identify prognostic transcriptomic expression patterns that characterize infection and organ dysfunction before the onset of clinical symptoms. Characteristic biomarker patterns were further identified, analysed and validated using quantitative RT-PCR on the Fluidigm BioMark^{™} real-time PCR array platform.

The Applicant conducted a large prospective, multicenter study in patients undergoing elective major surgery, with daily blood sampling and data recording commencing before the operation and continuing for up to a week after, to enable pre-symptomatic identification of patients developing infection complicated or not by new-onset organ dysfunction (sepsis). Crucially, there was a clinical adjudication panel who independently examined clinical and laboratory data to identify patients with definite infection (± sepsis). Samples from these patients enabled accurate comparison of microarray and RT-qPCR data against cohorts of age-, sex- and procedure-matched patients with non-infective systemic inflammation (SIRS) or an uncomplicated postoperative course. Nucleic acid expression signatures measured in blood samples could identify patients developing organ dysfunction, or infection, up to three days prior to clinical presentation, and could differentiate between patients developing uncomplicated infection or sepsis (organ dysfunction).

### Patient recruitment

Elective surgery patients were prospectively recruited into this study between November 2007 and February 2017. Patients were enrolled if they gave informed consent, were between 18-80 years of age and undergoing an elective high-risk surgical procedure that placed them at an increased risk of infection ± sepsis. Recruitment occurred at seven centers in the UK and one in Germany.

Clinical and blood samples were collected from 4,385 patients undergoing high-risk elective surgery. The total number of sample vials received was 72,734, and subsequent sub-aliquoting of key samples for further analysis generated a further 81,800 vials. 155 patients were adjudicated by the clinical advisory panel (CAP) to have definite post-operative infection. Samples from 63 of these patients, of whom 37 developed new organ dysfunction (sepsis), underwent detailed analyses. Samples from the first 58 of these infected patients were selected for microarray analysis with comparison made against 55 age-, sex- and procedure-matched patients with an uncomplicated, non-infected, non-inflamed course. 80

### Sampling

Blood sample collection occurred once between 1-7 days before surgery and then daily postoperatively until seven days, hospital discharge (if earlier), or diagnosis of infection or sepsis by the treating clinician.

### Sepsis patient selection process

Initial diagnosis of infection or sepsis was based on the treating clinician's interpretation of clinical and laboratory markers using the then-extant 'Sepsis-2' definition of sepsis. This described *'sepsis'* as suspected or confirmed infection with two or more systemic inflammatory response syndrome (SIRS) criteria, and *'severe sepsis'* as sepsis plus new-onset organ dysfunction. The new sepsis definition (Sepsis-3) published in February 2016 rebadged *'sepsis'* as infection plus new organ dysfunction identified by a rise of ≥2 points from one day to the next of the patient's SOFA score. To keep in line with modern nomenclature, subsequent analyses and descriptors apply the new definition.

As initial diagnosis of infection and sepsis is often based on clinical judgement and before any microbiological confirmation, the CAP was formed to adjudicate cases labelled as postoperative infection. A minimum of five specialists in intensive care or microbiology independently reviewed clinical, laboratory and imaging results to give a high confidence diagnosis of infection. These patients were allocated to either an infection group, a non-infective systemic inflammation (SIRS) group, or an uncomplicated post-operative recovery group assuming no significant non-infective issues arose (e.g. hemorrhage, myocardial infarction). The infection group was subsequently divided into 2 sub-groups, those with or without organ dysfunction as defined by an increase of the patient's SOFA score by 2 or more from one day to the next.

### Microarray analysis

Microarray analysis performed on blood samples taken up to 3 days (Days -1, -2 and -3) before clinical diagnosis of infection identified 80 transcripts that were taken through to qRT-PCR studies. Comparison was made against qRT-PCR measured on the same genes in age-, sex- and procedure-matched patients who either had non-infective systemic inflammation (n=58 ) or an uncomplicated recovery (n=62 , including the 55 patients used as the original controls).

For each sample analysed, globin-reduced RNA (GlobinClear^{™}, ThermoFisher) was prepared from total RNA. RNA integrity was measured using an Agilent Bioanalyzer 2100 (Town, State) and concentration using a NanoQuant^{™} (Tecan, Town). cRNA was prepared by amplification and labeling using the Illumina^{®} TotalPrep^{™} RNA Amplification Kit (ThermoFisher) and hybridized to Human HT-12v4 Beadarrays (Illumina^{®},Place, State). An Illumina^{®} HighScanHQ^{™} then imaged each chip with resulting intensities indicating the expression level of each probe's corresponding gene.

The Illumina^{®} Human HT-12v4 beadarrays were preprocessed and background corrected using GenomeStudio^{™} Software v2011.1 (Illumina^{®}). To obtain genes with the greatest evidence of differential expression, a linear model fit was applied for each gene using the limma package (Doi: 10.1093/nar/gkv007: Ritchie, M.E. et al, Nucleic Acids Res., 2015, 43(7)). Datasets include patient group with or without infection up to three days before diagnosis of infection. Data obtained from non-infected patients were used as a reference. A false positive rate of 0.05 with FDR correction and a fold change greater than 1.3 was taken as the level of significance.

### qPCR Methods and Analysis

### RNA Isolation

Total RNAs were extracted from an aliquot of 1,832 samples containing 400 µl whole blood immersed in 1 mL RNAlater using a twostep protocol as implemented in the RiboPure^{™}-Blood Kit (ThermoFisher Scientific). RNAlater was removed and the cells were re-suspended in 800 µl guanidinium-based lysis solution. After addition of 50 µl sodium acetate solution, RNA was extracted with 500 µl acid phenol/chloroform. After phase separation, the aqueous phase was recovered and 600 µl 100% ethanol added for binding of the RNA on a glass fiber filter spin column. After three wash steps the RNA was eluted in 2x 50 µl elution solution.

The purity and the concentration of the resulting RNA was determined with spectrophotometry on a Nanodrop^{™} 8000 and the RNA integrity was analyzed on an Agilent Bioanalyzer. The RNA samples were then normalized to 15 ng/µl using 96 well UF plates (Qiagen, Hilden, Germany).

### Quantitative Gene-Expression

For cDNA synthesis, 100 ng RNA was used as input in a 20 µl reaction using the High Capacity cDNA Kit (ThermoFisher Scientific) and the following incubation program; 25°C 10 min, 37°C 120 min, 85°C 5 min. For the standard curve, 1000 ng reference RNA was used as input in a 20 µl reaction. Negative controls were Non-Enzyme Control (NEC) prepared using 100 ng RNA without reverse transcriptase and Non-Template Control (NTC) prepared from a reverse transcription with RNase free water instead of RNA.

Specific Target Amplification (STA) reactions were performed in accordance with the Fluidigm Specific Target Amplification Quick Reference [PN 69000133 RevB]. In short, 1.25 µl cDNA was mixed with 2.5 µl TaqMan PreAmp Master Mix (2x) [PN 4391128] and 1.25 µl Pooled assay mix (0.2x) and amplified in a thermal cycler under the following conditions; 95°C 10 min pre-incubation followed by 14 cycles of 95°C 15 sec, 60°C 4 min.

Gene Expression Analysis was measured by multiplex real time PCR on a BioMark HD system using 96.96 Dynamic Arrays (Fluidigm, San Francisco, CA) and TaqMan Gene Expression Assays (Applied Biosystems, Carlsbad, CA) with three technical replicates. The Taqman assays for FAM105B and RANBP3 were used to normalize the relative abundance of transcripts between samples. The optimal set of reference genes was determined using geNorm analysis. The analysis showed that optimal normalization was achieved using the two genes FAM105B and RANBP3 (geNorm V < 0.15 when comparing a normalization factor based on the two or three most stable targets).

### Feature selection and learning of predictive models

To improve the performance metric of the predictive models a two-step feature selection was performed. First, the Boruta algorithm, a wrapper method based on Random forest was used for selection of relevant features in the data set. Then a new randomized feature (shadow feature) was added for each feature in the dataset. The classifier was then trained with the dataset and the importance of each feature calculated. Real features that have a significantly higher z-score than the best shadow feature are called relevant features. The Boruta algorithm was applied in a 5-fold crossvalidation, repeated 25 times. A feature identified as relevant in at least one model was considered for further evaluation. As a second step, backward elimination was used to determine those features with the most discriminatory power for a particular classification problem. Starting with all relevant features in a 5 fold cross validation repeated 25 times the importance of features was calculated.

This loop was reiterated until a maximum of the assessment index area under the curve (AUC) was found. In each iteration step, the feature with the least importance was removed.

### Results

### Microarray Gene Expression Analysis of Infected Patients

Transcriptomic sequencing was carried out on samples from 58 patients taken over the three days preceding clinical presentation of post-operative infection and 55 matched healthy postoperative controls. Overall 2337 differentially expressed genes (DEGs) with fold change of at least 1.2 between infection and control were identified. Of 1500 DEGs with the highest fold change, 58% (870 DEGs) were up-regulated and 42% (630) downregulated. The top 10 genes with the highest fold change for up- (ZDHHC19, TDRD9, SLC2A11, RETN, PFKFB2, OLFM4, MMP8, HPGD, GPR84, CD177_ and downregulation genes (CCN3, KLRB1, CXCL8, IFIT3, IFIT1, GZMK, FCER1A, CLC, CD3D, CCR3) included several immune defense-relevant genes. Functional enrichment analysis of the 1500 DEGs yielded immune relevant pathways involving primarily neutrophil and T cell activity. Several genes were related to multiple categories such as neutrophil and T cell related categories, lymphocyte differentiation and the immune response to pathogens.

### Classification of development of infection based on microarray expression and RT-qPCR data

### Microarray

A random forest-based algorithm was used to classify differential gene expression on Days -1, -2, or - 3 prior to infection diagnosis against their respective non-infected controls. Random forest reports the most important genes to reach performance next to statistical metrics.

The best identified classification for Day -1 (based on 54 infection plus and 51 infection minus samples) reached an Area under the ROC Curve (AUC) of 0.970 and a positive predictive value (PPV) of 0.931 for a set of eight genes *(ASNSD1, B4GALT5, BIRC5,* C11ORF1, *CR1, KIF18, LDLR, ZKSCAN1).* The best identified classification for Day -2 (based on 45 infection plus and 42 infection minus samples) achieved an AUC of 0.965 (PPV 0.926) using a different set of eight genes from the overall set *(ARID5B, B4GALT5, CD3E, CTDP1, HVCN1, P4HB, SLC2A11, ZNF195),* and the best classification for Day -3 (based on 35 infection plus and 33 infection minus samples) achieved an AUC of 0.952 (PPV 0.904) using again eight different genes *(B4GALT5, CTDP1, DOK3, GPR183, LDLR, NFKBIA, P4HB, SOS2).*

Classification of infection was repeated with a requirement for the same set of genes used by the random forest classifier for each day (Days -1, -2 and -3) prior to infection diagnosis. The best performing classifiers by random forest required 8 gene features and achieved AUC values of 0.947 for Day -1 (PPV 0.911), 0.910 for Day -2 (PPV 0.810), and 0.897 for Day -3 (PPV 0.810). Alongside genes *LDLR, B4GALT5, KIF1B* and *CTDP1* that were already featured in the separate day infection classification models, four additional genes *(GAS7, SLC26A6, HLA-DMB, EIF4G3)* were needed to achieve the reported results.

The identified 8 gene set from the random forest classification of infection versus no infection was validated using RT-qPCR data. The original sample size of 58 patients with infection and 55 controls was increased to 62 per group with new patient sample data that were unknown to the Random Forest classifier of microarray data. All genes showed highly significant differential expression for infection against controls and were comparable with gene expression as determined by the microarray analysis.

### RT-qPCR

To increase the number of gene feature candidates, the classification of infection based on microarray expression data was reiterated and gene features for near-optimal classification performance were monitored. This resulted in a set of 80 genes designated for RT-qPCR validation. Again, random forest based classification yielded excellent performance. Using individual gene sets for identifying infection on individual days prior to infection diagnosis (compared to a control, non-infected, non-inflamed group) generated AUC values of 0.951 (PPV 0.914) for Day -1 (based on 59 infection plus and 59 infection minus samples; ATP2A2, GALM, HLA-DMA, HLA-DOA, LDLR, SLC36A1, TRPM2), 0.962 (PPV 0.879) for Day -2 (based on 47 infection plus and 47 infection minus samples; ACTR6, CAPN15, CD247, HLA-DMB, LARP4B, LDLR, LGALS2, LSG1, METTL7B, TNFAIP3) and 0.921 (PPV 0.880) for Day -3 (based on 35 infection plus and 36 infection minus samples; AFF1, CAPN15, FKBP5, GALM, KLHL2, LDHA, LDLR, LEPROTL1, LETMD1, MAFG, RPS10, TCEA3) based on 7, 9 and 12 genes, respectively. With the requirement for the same gene set being used for each day of infection, AUC values of 0.908 (PPV 0.862) for Day -1, 0.907 for Day -2 (PPV 0.850) and 0.850 for Day -3 (PPV 0.798) were achieved with a seven gene set: *B4GALT5, AFF1, LDLR, ATXN7L3, LARP4B, SLC36A1, TRPM2.*

As the clinical diagnostic challenge usually comes from differentiating infected from non-infected causes of a systemic inflammatory response (SIRS) rather than non-inflamed, non-infected patients, RT-qPCR derived expression was also used to compare patients with postoperative infection versus a non-infected SIRS response (SIRS). Random forest based classification yielded AUC values ranging from 0.951 to 0.962 (PPV range 0.878-0.905) for Days -1 to -3 with gene sets of 12 (CFLAR, DENND4B, EIF4G3, FXBP5, GZMK, LDLR, MED13L, NME8, RPS14, SLC36A1, SLC41A3, SPATA13), eight (CFLAR, FKBP5, HVCN1, LDLR, NME8, SLC41A3, SPATA13, STOM) and eight (CFLAR, DENND4B, EIF4G3, FBXW2, MED13L, METTL7B, SPATA13, STOM), respectively. Using the same set of 12 genes (ATXN1, B4GALT5, CFLAR, EIF4G3, HVCN1, LDLR, MED13L, METTL7B, MIDN, SLC41A3, SPATA13, STOM) for each classification model regardless of the day prior to infection diagnosis, AUC values of 0.913 (Day -1), 0.938 (Day -2) and 0.927 (Day -3) were achieved.

### Classification of development of organ dysfunction (sepsis) versus non-complicated infection based on microarray expression and RT-qPCR data

### Microarray

A further random forest-based model set was generated using the microarray data for classifying infected patients either with development of organ dysfunction (sepsis) or without organ dysfunction (non-complicated). The best classification for Day -1 (based on 31 organ dysfunction plus and 23 non-complicated infection samples) achieved an AUC of 0.852 (PPV 0.785) based on seven genes *(HLA-DMA, HLA-DMB, ICAM2, INO80D, KIF1B*, *MED13L, QSOX1).* The best classification for Day -2 (based on 26 organ dysfunction plus and 19 non-complicated infection samples) was achieved with eight genes (*AFF1, BIN1, C19ORF70*, *GAS7, LDLR, MIDN, MRPS27, P4HB),* yielding an AUC of 0.853 (PPV 0.789). The classification performance for Day -3 (based on 20 organ dysfunction plus and 15 non-complicated infection samples) yielded an AUC of 0.951 (PPV 0.919) based on five genes (*C110RF1, CD3D, CTSS, LSG1, RPL13A).*

Following the same procedure as for development of infection, another classification model set based on random forest was created that required the same gene set for each separate day. AUC values of 0.783 (PPV 0.749) for Day -1, 0.724 (PPV 0.696) for Day -2 and 0.712 (PPV 0.738) were achieved with eight genes *(BIRCS, CPA3, DHRS3, HLA-DMA, ICAM2, MIDN, TNFAIP3, ZNF608).*

### RT-qPCR

Finally, RT-qPCR based expression was used for classification of sepsis versus non-complicated infection. Individual gene sets per day prior to infection diagnosis yielded AUC values of 0.884 (PPV 0.828) with 8 genes (based on 36 organ dysfunction plus and 23 non-complicated infection samples; AKR1B1, DOK3, ICAM2, IL1R1, RPL13A, RPS14, SGSH, TLR2), 0.850 ( PPV 0.788) with 12 genes (based on 28 organ dysfunction plus and 17 non-complicated infection samples; B3GNT5, C11ORF1, CD3E, IL1R1, LARP4B, LGALS2, LSG1, METTL7B, P4HB, SLC36A1, SOS2, STOM) and 0.883 (PPV 0.873) with 10 genes (based on 19 organ dysfunction plus and 16 non-complicated infection samples; CD247, CD3D, FCER1A, GRB10, IL1R1, LGALS2, RARRES3, RPS14, SGSH, TCEA3) for Days -1, -2 and -3, respectively. Requiring a common gene set for each day prior to infection diagnosis yielded AUC values of 0.869 (PPV 0.778) for Day -1, 0.774 (PPV 0.724) for Day -2 and 0.746 (PPV 0.706) for Day-3 based on an eight gene set *(SGSH, RPS13, DOK3, ICAM2, IL1R1, LGALS2, LSG1, RPL13A).*

To support a clinical transition, further models using all available patient samples were designed but this time all time points were combined to identify biomarker signatures for each question, whether prediction of infection or organ dysfunction, since in practice the day prior to infection and/or organ dysfunction would be unknown for a newly presented patient. Underlying RT-qPCR data were generated for the 25 nucleic acid markers present in the three biomarker signatures used for analysis and prediction for all days from the RT-PCR analysis, regardless of the day prior to clinical presentation (AFF1, ATXN1, ATXN7L3, B4GALT5, CFLAR, HVCN1, LDLR, LGALS2, LSG1, MED13L, RPS13, SGSH, SLC36A1, ICAM2, MIDN, METTL7B, LARP4B, EIF4G3, STOM, TRPM2, RPL13A, DOK3, SLC41A3, SPATA13, IL1R1; 7 nucleic acid signature for patients with infection versus no infection controls; 12 nucleic acid signature for patients with infection versus SIRS; 8 nucleic acid signature for organ dysfunction versus non-complicated infection).

### Classification of development of organ dysfunction (sepsis) versus all other clinical symptoms based on selected RT-qPCR data

A model was designed to classify patients presenting infections with new developed organ dysfunction (sepsis, n = 98) against patients showing any of the other presented symptoms (n = 357). The latter included infection without organ dysfunction, non-infected and inflamed (SIRS⁺), and non-infected and non-inflamed (SIRS⁻). Underlying RT-qPCR data were generated for the 25 nucleic acid markers present in each model for the same gene (nucleic acid) signature regardless of the day prior to clinical presentation. All time points were included per group and one model was sought for the classification of sepsis versus all other clinical symptoms. Using 22 nucleic acids (AFF1, ATXN1, ATXN7L3, B4GALT5, CFLAR, HVCN1, LDLR, LGALS2, LSG1, MED13L, RPS13, SGSH, SLC36A1, ICAM2, MIDN, METTL7B, LARP4B, EIF4G3, STOM, TRPM2, RPL13A, DOK3) an AUC value of 0.874 (PPV 0.760) was achieved to differentiate sepsis from all other clinical presentations. Requiring a reduced number of measured nucleic acids led to AUC classification performances of 0.866 (PPV 0.720) for 14 transcripts (AFF1, ATXN1, ATXN7L3, B4GALT5, CFLAR, HVCN1, LDLR, LGALS2, LSG1, MED13L, SGSH, MIDN, METTL7B, LARP4B), 0.841 (PPV 0.655) for six nucleic acids (ATXN7L3, B4GALT5, LDLR, MED13L, ATXN1, CFLAR) and 0.810 (PPV 0.579) for four nucleic acids (ATXN7L3, B4GALT5, LDLR, MED13L).

Another model design was also employed, where the patient data was split prior to the training such that 10% of the data were never seen by the classifier. Selecting randomly 20 times 10% testing data and repeating the same model training procedure as in the prior classifications for the remaining 90% resulted in an average classification AUC of 0.830 (PPV 0.665), for a thirteen gene nucleic acid set, or biomarker signature, comprising *AFF1, ATXN1, ATXN7L3, B4GALT5, CFLAR, HVCN1, LDLR, LGALS2, LSG1, MED13L, RPS13, SGSH* and *SLC36A1.*

### Classification of development of infection versus non-infected and non-inflamed (SIRS⁻) based on selected RT-qPCR data

A model was designed to classify patients presenting infections with new developed infection ( n = 153) against non-infected and non-inflamed patients (n = 151). Underlying RT-qPCR data were generated for the 25 nucleic acid markers present in each model for the same gene (nucleic acid) set regardless of the day prior to clinical presentation. All time points were included per group and one model was sought for the classification of infection. Using 21 nucleic acids (LDLR, SGSH, AFF1, ATXN7L3, HVCN1, LGALS2, LSG1, MED13L, RPS13, ATXN1, B4GALT5, CFLAR, DOK3, ICAM2, LARP4B, METTL7B, MIDN, RPL13A, SLC36A1, STOM, TRPM2) an AUC value of 0.897 (PPV 0.810) was achieved to predict infection. Requiring a reduced number of measured nucleic acids led to AUC classification performances of 0.891 (PPV 0.812) for 9 transcripts (LDLR, SGSH, AFF1, ATXN7L3, HVCN1, LGALS2, LSG1, MED13L, RPS13), and 0.810 (PPV 0.730) for two nucleic acids (LDLR, SGSH).

Another model design was also employed, where the patient data was split prior to the training such that 10% of the data were never seen by the classifier. Selecting randomly 20 times 10% testing data and repeating the same model training procedure as in the prior classifications for the remaining 90% resulted in an average classification AUC of 0.895 (PPV 0.815), for a thirteen gene nucleic acid set, or biomarker signature, comprising AFF1, ATXN1, ATXN7L3, B4GALT5, LARP4B, LDLR, MED13L, METTL7B, MIDN, RPS13, SGSH, LC36A1, TRPM2.

### Classification of development of infection versus non-infected and inflamed (SIRS⁺) based on selected RT-qPCR data

A model was designed to classify patients presenting infections with new developed infection (n = 153) against non-infected and inflamed patients (n = 148). Underlying RT-qPCR data were generated for the 25 nucleic acid markers present in each model for the same gene (nucleic acid) set regardless of the day prior to clinical presentation. All time points were included per group and one model was sought for the classification of infection. Using 25 nucleic acids (ATXN1, ATXN7L3, CFLAR, B4GALT5, EIF4G3, HVCN1, LARP4B, MED13L, METTL7B, MIDN, SLC36A1, SPATA13, AFF1, DOK3, ICAM2, IL1R1, LDLR, LGALS2, LSG1, RPL13A, RPS13, SGSH, SLC41A3, STOM, TRPM2) an AUC value of 0.935 (PPV 0.866) was achieved to predict infection. Requiring a reduced number of measured nucleic acids led to AUC classification performances of 0.940 (PPV 0.863) for 12 transcripts (ATXN1, ATXN7L3, CFLAR, B4GALT5, EIF4G3, HVCN1, LARP4B, MED13L, METTL7B, MIDN, SLC36A1, SPATA13), and 0.888 (PPV 0.826) for three nucleic acids (ATXN1, ATXN7L3, CFLAR).

Another model design was also employed, where the patient data was split prior to the training such that 10% of the data were never seen by the classifier. Selecting randomly 20 times 10% testing data and repeating the same model training procedure as in the prior classifications for the remaining 90% resulted in an average classification AUC of 0.915 (PPV 0.843), for a nine gene nucleic acid set, or biomarker signature, comprising ATXN1, ATXN7L3, B4GALT5, CFLAR, EIF4G3, HVCN1, MED13L, METTL7B, MIDN.

### Classification of development of organ dysfunction versus no organ dysfunction based on selected RT-qPCR data

A model was designed to classify patients presenting infections with new developed infection (n = 161) against no organ dysfunction (n = 291). Underlying RT-qPCR data were generated for the 25 nucleic acid markers present in each model for the same gene (nucleic acid) set regardless of the day prior to clinical presentation. All time points were included per group and one model was sought for the classification of infection. Using 23 nucleic acids (AFF1, HVCN1, LDLR, LGALS2, LSG1, SGSH, ATXN7L3, MED13L, METTL7B, RPS13, STOM, ATXN1, B4GALT5, CFLAR, DOK3, ICAM2, LARP4B, MIDN, RPL13A, SLC36A1, SLC41A3, SPATA13, TRPM2) an AUC value of 0.744 (PPV 0.666) was achieved to predict organ dysfunction. Requiring a reduced number of measured nucleic acids led to AUC classification performances of 0.750 (PPV 0.632) for 11 transcripts (AFF1, HVCN1, LDLR, LGALS2, LSG1, SGSH, ATXN7L3, MED13L, METTL7B, RPS13, STOM), and 0.717 (PPV 0.623) for six nucleic acids (AFF1, HVCN1, LDLR, LGALS2, LSG1, SGSH).

Another model design was also employed, where the patient data was split prior to the training such that 10% of the data were never seen by the classifier. Selecting randomly 20 times 10% testing data and repeating the same model training procedure as in the prior classifications for the remaining 90% resulted in an average classification AUC of 0.672 (PPV 0.538), for a nine gene nucleic acid set, or biomarker signature, comprising ATXN1, ATXN7L3, B4GALT5, CFLAR, EIF4G3, HVCN1, MED13L, METTL7B, MIDN.

Overall 80 nucleic acid markers were identified in the study as being relevant and highly significant to the prediction of development of infection and/or development of organ dysfunction. The 80 nucleic acid markers are:
ACTR6, AFF1, AKR1B1, ARID5B, ASNSD1, ATP2A2, ATXN1, ATXN7L3, B3GNT5, B4GALT5, BIN1, BIRCS, C11ORF1, C19ORF70, CAPN15, CD247, CD3D, CD3E, CFLAR, CPA3, CR1, CTDP1, CTSS, DENND4B, DHRS3, DOK3, EIF4G3, FBXW2, FCER1A, FKBP5, GALM, GAS7, GPR183, GRB10, GZMK, HLA-DMA, HLA-DMB, HLA-DOA, HVCN1, ICAM2, IL1R1, INO80D, KIF1B, KLHL2, LARP4B, LDHA, LDLR, LEPROTL1, LETMD1, LGALS2, LSG1, MAFG, MED13L, METTL7B, MIDN, MRPS27, NFKBIA, NME8, P4HB, QSOX1, RARRES3, RPL13A, RPS10, RPS13, RPS14, SGSH, SLC26A6, SLC2A11, SLC36A1, SLC41A3, SOS2, SPATA13, STOM, TCEA3, TLR2, TNFAIP3, TRPM2, ZKSCAN1, ZNF195, ZNF608.

Of these 80 nucleic acid markers, 47 markers are particularly relevant for predicting infection, as compared to controls:
ACTR6, AFF1, ARID5B, ASNSD1, ATP2A2, ATXN7L3, B4GALT5, BIRC5, C11ORF1, CAPN15, CD247, CD3E, CR1, CTDP1, DOK3, EIF4G3, FKBP5, GALM, GAS7, GPR183, HLA-DMA, HLA-DMB, HLA-DOA, HVCN1, KIF1B, KLHL2, LARP4B, LDHA, LDLR, LEPROTL1, LETMD1, LGALS2, LSG1, MAFG, METTL7B, NFKBIA, P4HB, RPS10, SLC26A6, SLC2A11, SLC36A1, SOS2, TCEA3, TNFAIP3, TRPM2, ZKSCAN1, ZNF195.

Of these 80 nucleic acid markers, 45 markers are particularly relevant for predicting organ dysfunction, as compared to patients developing infection alone (non-complicated infection):
AFF1, AKR1B1, B3GNT5, BIN1, BIRC5, C11ORF1, C19ORF70, CD247, CD3D, CD3E, CPA3, CTSS, DHRS3, DOK3, FCER1A, GAS7, GRB10, HLA-DMA, HLA-DMB, ICAM2, IL1R1, INO80D, KIF1B, LARP4B, LDLR, LGALS2, LSG1, MED13L, METTL7B, MIDN, MRPS27, P4HB, QSOX1, RARRES3, RPL13A, RPS13, RPS14, SGSH, SLC36A1, SOS2, STOM, TCEA3, TLR2, TNFAIP3, ZNF608.

Of these 80 nucleic acid markers, 19 are particularly relevant for predicting infection, as compared to patients developing SIRS criteria:
ATXN1, B4GALT5, CFLAR, DENND4B, EIF4G3, FBXW2, FKBPS, GZMK, HVCN1, LDLR, MED13L, METTL7B, MIDN, NME8, RPS14, SLC36A1, SLC41A3, SPATA13, STOM

The Applicant has interrogated this nucleic acid marker data, in light of specifically effective subsets for predicting infection or organ dysfunction, and concluded that subsets of 4 or 5 nucleic acid markers (and potentially less) from the 80, 47, 45, or 19, should be capable of predicting infection or organ dysfunction with high AUCs, or accuracy.

Markers that occur in multiple exemplified subsets are more likely to provide further effective down-selected subsets, and thus the Applicant suggests recurring nucleic acid markers should be combined to provide further subsets for predicting infection and/or organ dysfunction. Key markers include especially LDLR, AFF1, CAPN15, GALM, LARP4B, SLC36A1, and ZKCSAN1 (for especially differentiating infection from non-infection), IL1R1, LGALS2, SGSH, DOK3, ICAM2, LSG1, RPL13A, and RPS14 (for especially differentiating organ dysfunction plus infection from non-complicated infection (without organ dysfunction)), though other genes occurring in multiple down-selected marker sets include HLA-DMA, METTL7B, TNFAIP3, LDHA, TCEA3, BIRC5, EIF4G3, and B4GALT5 (especially for infection versus non-infection), QSOX1, MIDN and CD247 (for especially differentiating organ dysfunction plus infection from non-complicated infection). A suitable marker set for infection versus no infection may include for example LDLR, which occurs in all down-selected lists from qRT-PCR analysis, and for organ dysfunction may include IL1R1, which occurs in all down-selected lists from qRT-PCR analysis, perhaps with LGALS2 and SGSH, which occur in the majority of down-selected lists from qRT-PCR analysis.

This study is the culmination of over 10 years of research into the pathogenesis of sepsis in elective surgery patients. Analysis of the host transcriptome in whole blood samples before and after surgery has led to the identification of a number of key host biomarkers whose expression enables differentiation of sepsis patients from other cohorts. Whilst whole transcriptome studies of sepsis have been widely reported, all have focussed on patients with established symptoms i.e. patients diagnosed with sepsis or infection before sampling began. However, these have limited utility for the early diagnosis of infection, or pre-symptomatic prediction of infection or organ dysfunction. In addition, the complicated nature of whole transcriptomic data has limited its clinical utility for a number of practical and interpretational reasons.

The work reported here is unique since it has sought to address these two issues. Firstly, it has described the early host response that leads to sepsis through characterisation of the transcriptome of patients that go on to develop sepsis. Secondly, it has down-selected genes capable of discriminating between infection, organ dysfunction (sepsis) and other patient cohorts, and proved that it is possible to identify clinically useful host biomarker signatures (with low numbers of markers, less than twenty, and often less than ten required to provide a prediction) to predict infection and organ dysfunction in advance of symptoms.

In order to understand the host response that leads to sepsis, a unique approach to patient recruitment was adopted. Clarity on the provenance of each patient was considered as important as the clinical data itself for robust modelling of early sepsis pathogenesis. Patients were comparatively well when they entered the study. Apart from the underlying need for surgery, patients were infection free and were in relatively good health. This was underlined by the high rates of uncomplicated recovery observed in the study. However, 3.53% of the patients recruited into this study did develop organ dysfunction (sepsis). The prospective collection of samples before and after surgery enabled the detailed characterisation of changes in gene expression that led to the development of infection or sepsis in this elective surgery cohort. The low incidence of sepsis also gave a large patient cohort from which age/sex/procedure matched patients could be selected for inter-patient comparison. This enabled the effects of age, gender and surgical procedure to be controlled effectively.

Successful down-selection of genes to manageable numbers is vital for transition to a platform. Consequently, a machine learning algorithm approach has been used to select appropriate targets and classify patients based on host gene expression with output compared to clinical diagnosis to determine predictive accuracy. The success of this approach is evidenced by high AUC values and small biomarker signatures (subsets of nucleic acid markers) of between 4 or 5 markers, up to about 12 markers or more (though generally less than twenty), when comparing infection and comparator, infection and SIRS and sepsis (organ dysfunction⁺) with infection only patients. These biomarker signatures are significantly much smaller than previously reported signatures for pre-symptomatic prediction of infection, and especially for pre-symptomatic prediction of organ dysfunction.

In trying to answer the more difficult question of when an individual will develop sepsis, this study was able to utilise multiple sample time points collected from each patient.

## Claims

1. A method for predicting or monitoring the development of infection, versus the development of systemic inflammatory response (SIRS), in a subject, the method comprising determining levels of nucleic acid markers selected from the list consisting of: ATXN1, B4GALT5, CFLAR, DENND4B, EIF4G3, FBXW2, FKBP5, GZMK, HVCN1, LDLR, MED13L, METTL7B, MIDN, NME8, RPS14, SLC36A1, SLC41A3, SPATA13, and STOM in a biological sample taken from the subject, wherein the nucleic acid markers at least comprise ATXN1, B4GALT5, CFLAR, EIF4G3, HVCN1, LDLR, MED13L, METTL7B, MIDN, SLC41A3, SPATA13, STOM;
wherein the levels of the markers are used to predict or monitor the development of infection, versus development of SIRS, in the subject.

2. Method according to Claim 1 wherein the nucleic acid markers consist of: ATXN1, B4GALT5, CFLAR, DENND4B, EIF4G3, FBXW2, FKBP5, GZMK, HVCN1, LDLR, MED13L, METTL7B, MIDN, NME8, RPS14, SLC36A1, SLC41A3, SPATA13, and STOM.

3. A method according to Claim 1 or Claim 2, wherein the method is computer implemented using an appropriate mathematical model to analyse data generated.

4. A kit for predicting or monitoring development of infection in a subject, said kit consisting of reagents and/or systems for specifically determining levels of nucleic acid markers selected from the list consisting of: ATXN1, B4GALT5, CFLAR, DENND4B, EIF4G3, FBXW2, FKBP5, GZMK, HVCN1, LDLR, MED13L, METTL7B, MIDN, NME8, RPS14, SLC36A1, SLC41A3, SPATA13, and STOM in a biological sample taken from the subject, wherein the nucleic acid markers at least comprise ATXN1, B4GALT5, CFLAR, EIF4G3, HVCN1, LDLR, MED13L, METTL7B, MIDN, SLC41A3, SPATA13, STOM.

5. A kit according to Claim 4, wherein the reagents and/or systems for specifically determining levels of the nucleic acid markers comprise reagents for nucleic acid amplification, which reagents for nucleic acid amplification comprise one or more of fluorescently-labelled oligonucleotide probes or fluorescently-labelled primers, wherein the fluorescently-labelled oligonucleotide probes or fluorescently-labelled primers consist of probes and primers each capable of specific binding to nucleic acid products of the nucleic acid markers, when the nucleic acid amplification is a real-time (RT) polymerase chain reaction (PCR).

6. An apparatus for analysis of a biological sample from a subject to predict or monitor the development of infection in the subject consisting means for specifically monitoring, measuring or detecting the expression of nucleic acids selected from the list consisting of:
ATXN1, B4GALT5, CFLAR, DENND4B, EIF4G3, FBXW2, FKBP5, GZMK, HVCN1, LDLR, MED13L, METTL7B, MIDN, NME8, RPS14, SLC36A1, SLC41A3, SPATA13, and STOM in a biological sample from the subject, wherein the nucleic acid markers at least comprise ATXN1, B4GALT5, CFLAR, EIF4G3, HVCN1, LDLR, MED13L, METTL7B, MIDN, SLC41A3, SPATA13, STOM; and means for analysis of data produced from the means for specifically monitoring, measuring or detecting, and means for providing an output from the analysis which output provides a prediction of the likelihood of a subject having an infection, or an output to enable monitoring of infection.

7. An apparatus according to Claim 6, wherein the means for analysis of data is computer implemented using an appropriate mathematical model to analyse the data.

## Patentansprüche

1. Verfahren zum Vorhersagen oder Überwachen der Entwicklung einer Infektion im Vergleich zur Entwicklung einer systemischen Entzündungsreaktion (SIRS) bei einem Patienten, wobei das Verfahren das Bestimmen der Konzentrationen von Nukleinsäuremarkern, die aus der Liste bestehend aus ATXN1, B4GALT5, CFLAR, DENND4B, EIF4G3, FBXW2, FKBP5, GZMK, HVCN1, LDLR, MED13L, METTL7B, MIDN, NME8, RPS14, SLC36A1, SLC41A3, SPATA13 und STOM ausgewählt sind, in einer dem Patienten entnommenen biologischen Probe umfasst, wobei die Nukleinsäuremarker mindestens ATXN1, B4GALT5, CFLAR, EIF4G3, HVCN1, LDLR, MED13L, METTL7B, MIDN, SLC41A3, SPATA13 und STOM umfassen;
wobei die Konzentrationen der Marker verwendet werden, um die Entwicklung einer Infektion im Vergleich zur Entwicklung einer SIRS bei dem Patienten vorherzusagen oder zu überwachen.

2. Verfahren nach Anspruch 1, bei dem die Nukleinsäuremarker bestehen aus ATXN1, B4GALT5, CFLAR, DENND4B, EIF4G3, FBXW2, FKBP5, GZMK, HVCN1, LDLR, MED13L, METTL7B, MIDN, NME8, RPS14, SLC36A1, SLC41A3, SPATA13 und STOM.

3. Verfahren nach Anspruch 1 oder Anspruch 2, wobei das Verfahren unter Verwendung eines geeigneten mathematischen Modells zur Analyse der generierten Daten computerimplementiert ist.

4. Kit zur Vorhersage oder Überwachung der Entwicklung einer Infektion bei einem Patienten, wobei das Kit aus Reagenzien und/oder Systemen zur spezifischen Bestimmung der Konzentrationen von Nukleinsäuremarkern, die ausgewählt sind aus der Liste bestehend aus ATXN1, B4GALT5, CFLAR, DENND4B, EIF4G3, FBXW2, FKBP5, GZMK, HVCN1, LDLR, MED13L, METTL7B, MIDN, NME8, RPS14, SLC36A1, SLC41A3, SPATA13 und STOM in einer dem Patienten entnommenen biologischen Probe besteht, wobei die Nukleinsäuremarker mindestens ATXN1, B4GALT5, CFLAR, EIF4G3, HVCN1, LDLR, MED13L, METTL7B, MIDN, SLC41A3, SPATA13 und STOM umfassen.

5. Kit nach Anspruch 4, bei dem die Reagenzien und/oder Systeme zur spezifischen Bestimmung der Konzentrationen der Nukleinsäuremarker Reagenzien zur Nukleinsäureamplifikation umfassen, wobei die Reagenzien zur Nukleinsäureamplifikation eine oder mehrere fluoreszenzmarkierte Oligonukleotid-Sonden oder fluoreszenzmarkierte Primer umfassen, wobei die fluoreszenzmarkierten markierten Oligonukleotid-Sonden oder fluoreszenzmarkierten Primer aus Sonden und Primern bestehen, die jeweils in der Lage sind, spezifisch an Nukleinsäureprodukte der Nukleinsäuremarker zu binden, wenn die Nukleinsäureamplifikation eine Echtzeit-Polymerasekettenreaktion (RT-PCR) ist.

6. Vorrichtung zur Analyse einer biologischen Probe von einem Patienten, um die Entwicklung einer Infektion in dem Patienten vorherzusagen oder zu überwachen, bestehend aus Mitteln zum spezifischen Überwachen, Messen oder Nachweisen der Expression von Nukleinsäuren, ausgewählt aus der Liste bestehend aus: ATXN1, B4GALT5, CFLAR, DENND4B, EIF4G3, FBXW2, FKBP5, GZMK, HVCN1, LDLR, MED13L, METTL7B, MIDN, NME8, RPS14, SLC36A1, SLC41A3, SPATA13 und STOM in einer von dem Patienten genommenen biologischen Probe, wobei die Nukleinsäuremarker mindestens ATXN1, B4GALT5, CFLAR, EIF4G3, HVCN1, LDLR, MED13L, METTL7B, MIDN, SLC41A3, SPATA13, STOM umfassen; Mitteln zur Analyse der von den Mitteln zum spezifischen Überwachen, Messen oder Nachweisen erzeugten Daten, und Mitteln zum Bereitstellen eines Ergebnisses der Analyse, wobei das Ergebnis eine Vorhersage der Wahrscheinlichkeit liefert, dass ein Patient eine Infektion hat, oder eines Ergebnisses, das die Überwachung einer Infektion ermöglicht.

7. Vorrichtung nach Anspruch 6, bei der die Mittel zur Analyse von Daten computerimplementiert sind und ein geeignetes mathematisches Modell zur Analyse der Daten verwenden.

## Revendications

1. Procédé pour prédire ou surveiller le développement d'une infection, *versus* le développement d'une réponse inflammatoire systémique (SRIS), chez un sujet, le procédé comprenant la détermination de niveaux de marqueurs d'acide nucléique sélectionnés parmi la liste constituée de : ATXN1, B4GALT5, CFLAR, DENND4B, EIF4G3, FBXW2, FKBP5, GZMK, HVCN1, LDLR, MED13L, METTL7B, MIDN, NME8, RPS14, SLC36A1, SLC41A3, SPATA13, et STOM dans un échantillon biologique prélevé sur le sujet, dans lequel les marqueurs d'acide nucléique comprennent au moins ATXN1, B4GALT5, CFLAR, EIF4G3, HVCN1, LDLR, MED13L, METTL7B, MIDN, SLC41A3, SPATA13, STOM ;
dans lequel les niveaux des marqueurs sont utilisés pour prédire ou surveiller le développement d'une infection, *versus* le développement du SRIS, chez le sujet.

2. Procédé selon la revendication 1, dans lequel les marqueurs d'acide nucléique sont constitués de : ATXN1, B4GALT5, CFLAR, DENND4B, EIF4G3, FBXW2, FKBP5, GZMK, HVCN1, LDLR, MED13L, METTL7B, MIDN, NME8, RPS14, SLC36A1, SLC41A3, SPATA13, et STOM.

3. Procédé selon la revendication 1 ou la revendication 2, dans lequel le procédé est mis en œuvre par ordinateur à l'aide d'un modèle mathématique approprié pour analyser les données générées.

4. Kit pour prédire ou surveiller le développement d'une infection chez un sujet, ledit kit étant constitué de réactifs et/ou de systèmes pour déterminer spécifiquement des niveaux de marqueurs d'acide nucléique sélectionnés parmi la liste constituée de : ATXN1, B4GALT5, CFLAR, DENND4B, EIF4G3, FBXW2, FKBP5, GZMK, HVCN1, LDLR, MED13L, METTL7B, MIDN, NME8, RPS14, SLC36A1, SLC41A3, SPATA13, et STOM dans un échantillon biologique prélevé sur le sujet, dans lequel les marqueurs d'acide nucléique comprennent au moins ATXN1, B4GALT5, CFLAR, EIF4G3, HVCN1, LDLR, MED13L, METTL7B, MIDN, SLC41A3, SPATA13, STOM.

5. Kit selon la revendication 4, dans lequel les réactifs et/ou systèmes pour déterminer spécifiquement des niveaux des marqueurs d'acide nucléique comprennent des réactifs pour l'amplification d'acide nucléique, lesquels réactifs pour l'amplification d'acide nucléique comprennent une ou plusieurs sondes oligonucléotidiques marquées par fluorescence ou amorces marquées par fluorescence, dans lequel les sondes oligonucléotidiques marquées par fluorescence sont constituées de sondes et d'amorces capables de se lier spécifiquement aux produits d'acide nucléique des marqueurs d'acide nucléique, lorsque l'amplification d'acide nucléique est une réaction en chaîne par polymérase (PCR) en temps réel (RT).

6. Appareil pour analyser un échantillon biologique provenant d'un sujet pour prédire ou surveiller le développement d'une infection chez ce sujet, consistant en un moyen pour surveiller, mesurer ou détecter spécifiquement l'expression d'acides nucléiques sélectionnés parmi la liste constituée de : ATXN1, B4GALT5, CFLAR, DENND4B, EIF4G3, FBXW2, FKBP5, GZMK, HVCN1, LDLR, MED13L, METTL7B, MIDN, NME8, RPS14, SLC36A1, SLC41A3, SPATA13, et STOM dans un échantillon biologique prélevé sur le sujet, dans lequel les marqueurs d'acide nucléique comprennent au moins ATXN1, B4GALT5, CFLAR, EIF4G3, HVCN1, LDLR, MED13L, METTL7B, MIDN, SLC41A3, SPATA13, STOM ; et un moyen pour analyser des données produites par le moyen pour surveiller, mesurer ou détecter spécifiquement, et un moyen pour fournir une sortie à partir de l'analyse, laquelle sortie fournit une prédiction de la probabilité qu'un sujet ait une infection, ou une sortie pour permettre la surveillance de l'infection.

7. Appareil selon la revendication 6, dans lequel le moyen pour analyser des données est mis en œuvre par ordinateur à l'aide d'un modèle mathématique approprié pour analyser les données.
